# EUROPEAN PATENT SPECIFICATION

(11) **EP 4 161 611 B1**
(45) Date of publication and mention of the grant of the patent: **08.04.2026**
(21) Application number: 21724342.7
(22) Date of filing: 14.05.2021
(51) Int. Cl.: A61M 5/42, A61M 5/31, A61M 5/32

(54) **MEDICAMENT DELIVERY DEVICE COMPONENT OR ADD-ON MODULE FOR MEDICAMENT DELIVERY DEVICE**
KOMPONENTE EINER MEDIKAMENTENABGABEVORRICHTUNG ODER ZUSATZEINHEIT FÜR MEDIKAMENTENABGABEVORRICHTUNG
COMPOSANT DE DISPOSITIF D'ADMINISTRATION DE MÉDICAMENTS OU DISPOSITIF COMPLÉMENTAIRE POUR DISPOSITIFS D'ADMINISTRATION DE MÉDICAMENTS

(30) Priority: 04.06.2020 EP 20178240
(43) Date of publication of application: 12.04.2023
(73) Proprietor: SHL Medical AG, 6302 Zug (CH)
(72) Inventor: CARPENTER, Lucas, Taoyuan City, 338 (TW)
(86) International application number: PCT/EP2021/062849
(87) International publication number: WO 2021/244835

(56) References cited:
- EP-A1- 2 886 148
- WO-A1-01/93931
- JP-A- 2012 239 824
- US-A1- 2007 021 716
- US-A1- 2015 258 284
- US-A1- 2018 326 163

## Description

### TECHNICAL FIELD

The invention concerns medicament delivery device components or medicament delivery device add-ons, and particularly concerns medicament delivery device components or medicament delivery device add-ons for alleviating injection pain.

### BACKGROUND

Although medicament delivery devices such as pen injectors and autoinjectors can make a significant positive difference to the well-being of patients and caregivers that use them, they can still suffer from the drawback of causing pain at the injection site. The applicant has appreciated that there is further potential for alleviation of this pain.

US2018/0326163 describes an attachment for an injection pen or syringe. The attachment has an array of inwardly pointing flexible fingers configured to gather a pucker of subcutaneous tissue in the vicinity of a needle insertion point.

US2007/0021716 describes a nozzle device suitable for use in jet injection and providing means for stretching the skin.

US2015/0258284 describes an injector such as a needle-type auto-injector having a flexible flange for stretching or pinching the skin of the injection site.

WO01/93931 describes a stretching device for stretching the skin to enhance contact by a medical device.

JP2012239824 describes an application tool for applying a cosmetic cream to the skin. A plurality of skin stretch pieces may be connected to a pressing surface via hinges.

EP2886148 describes a drug delivery aid comprising a substantially flat contact element with a second surface adapted to be placed on an injection site. The second surface may have a surface structure adapted to stimulate nerves on the skin.

### SUMMARY

The invention is defined by the appended claims, to which reference should now be made.

In the present disclosure, when the term "distal direction" is used, this refers to the direction pointing away from the dose delivery site during use of the medicament delivery device. When the term "distal part/end" is used, this refers to the part/end of the delivery device, or the parts/ends of the members thereof, which under use of the medicament delivery device is/are located furthest away from the dose delivery site. Correspondingly, when the term "proximal direction" is used, this refers to the direction pointing towards the dose delivery site during use of the medicament delivery device. When the term "proximal part/end" is used, this refers to the part/end of the delivery device, or the parts/ends of the members thereof, which under use of the medicament delivery device is/are located closest to the dose delivery site.

Further, the terms "longitudinal", "longitudinally", "axially" and "axial" refer to a direction extending from the proximal end to the distal end and along the device or components thereof, typically in the direction of the longest extension of the device and/or component.

Similarly, the terms "transverse", "transversal" and "transversally" refer to a direction generally perpendicular to the longitudinal direction.

A first aspect of the invention concerns a device for alleviating injection pain, the device comprising an attachment portion for attachment to a medicament delivery device, a flexible arm extending from a first end attached to the attachment portion to a second end distal from the attachment portion, and a plurality of protrusions attached to the flexible arm. Preferably, the device is a medicament delivery device component or a medicament delivery device add-on. Such a device can reduce injection (drug delivery) discomfort. In particular, such a device can reduce injection pain by skin stimulation, distracting from the injection and reducing the subjective pain and/or discomfort of injection, particularly during the injection itself.

Preferably, the width of the second end of the flexible arm is thinner than the width of the first end of the flexible arm. Preferably, the width of the flexible arm tapers from the first end to the second end. The flexible arm is more flexible at the second end than at the first end. Such configurations can result in provision of an increasing pressure applied by the device around the injection site as the device is pushed towards the injection site.

Preferably the protrusions are distributed along the flexible arm between the first end and the second end, for example along at least half of the length of the flexible arm, or more preferably along at least three-quarters of the length of the flexible arm. Preferably, the protrusions proximal to the first end of the flexible arm are longer than the protrusions proximal to the second end of the flexible arm. Such configurations can result in provision of an increased sensation applied by the device around the injection site as the device is pushed towards the injection site.

Preferably, the device extends along an axis from a proximal end to a distal end, wherein the first end of the flexible arm is closer to the distal end than the second end of the flexible arm. Preferably, the second end of the flexible arm is further from an axis than the first end of the flexible arm. Preferably, wherein the flexible arm describes an arc. Such configurations can help the arms spread reliably as the device is pushed towards the injection site.

Preferably, the device comprises a plurality of flexible arms, each flexible arm comprising a plurality of protrusions. This can provide a greater effect than a single arm. Preferably, the device is a medicament delivery device add-on and the attachment portion is removably attachable to a medicament delivery device.

A second aspect of the invention concerns a medicament delivery device comprising the device described above. Preferably, the flexible arm is closer than the attachment portion to a proximal end of the medicament delivery device.

Generally, all terms used in the claims are to be interpreted according to their ordinary meaning in the technical field, unless explicitly defined otherwise herein. All references to a/an/the element, apparatus, member, component, means, etc. are to be interpreted openly as referring to at least one instance of the element, apparatus, member component, means, etc., unless explicitly stated otherwise.

### BRIEF DESCRIPTION OF THE DRAWINGS

Embodiments of the invention will now be described by way of example only and with reference to the accompanying drawings, in which:
Figure 1 shows an example device.
Figure 2 shows a side view of one embodiment of the device of Figure 1.
Figure 3 shows a side view of another embodiment of the device of Figure 1.
Figure 4 shows a side view of an autoinjector comprising the device of Figure 1 before carrying out an injection.
Figure 5 shows a side view of the autoinjector of Figure 4 whilst carrying out an injection.

### DETAILED DESCRIPTION

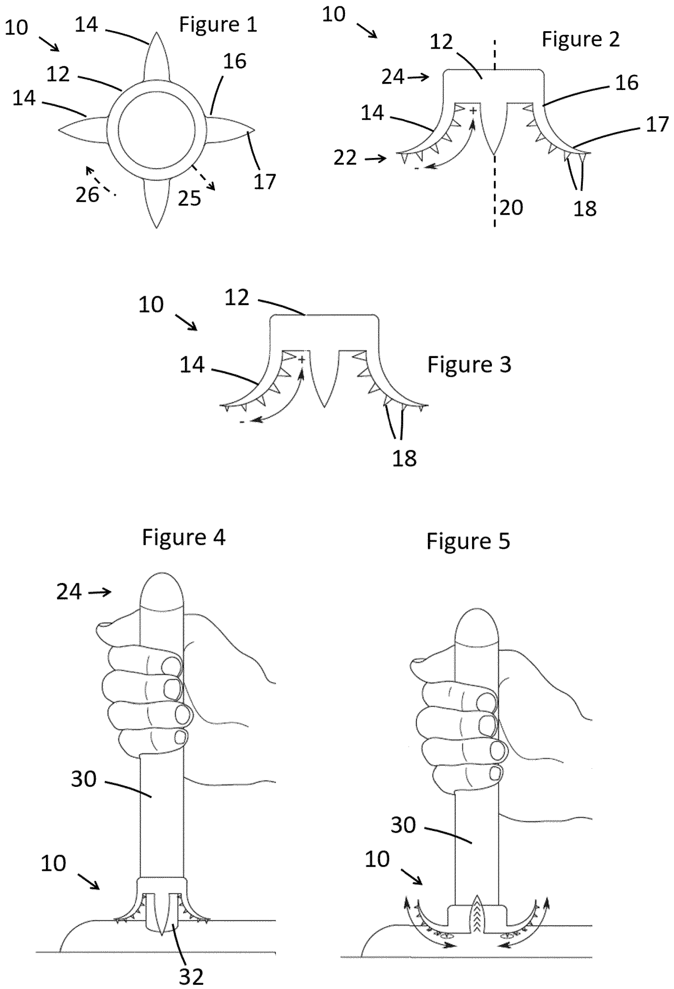

Figures 1 and 2 show a device 10 for alleviating injection pain, the device comprising an attachment portion 12 for attachment to a medicament delivery device, a flexible arm 14 extending from a first end 16 attached to the attachment portion to a second end 17 distal from the attachment portion, and a plurality of protrusions 18 attached to the flexible arm. The device is a medicament delivery device component or a medicament delivery device add-on.

Figure 2 shows the device 10 relative to an axis 20 (central axis), with the device 10 extending from a proximal end 22 to a distal end 24. In Figure 2, the protrusions are all the same size. Figure 3 shows a similar device, but with protrusions of varying sizes, with protrusions closer to the attachment portion being larger than protrusions further from the attachment portion.

Figures 4 and 5 show the device 10 attached to a medicament delivery device, in this case an autoinjector 30. A medicament delivery member shield 32 of the autoinjector can also be seen. Figure 4 shows the autoinjector prior to injection and Figure 5 shows the autoinjector during injection.

To use the device 10, the device is first attached to a medicament delivery device, for example by an end user prior to use of a medicament delivery device or by a manufacturer during manufacture of the medicament delivery device, particularly where the device is a medicament delivery device component. An injection can then be carried out as normal with the medicament delivery device. As the device is pressed against the skin, the flexible arms flex outwards away from the axis 20, and the protrusions 18 rub across the skin, providing a sensation that can distract from injection pain. Once an injection has been completed, the medicament delivery device can be removed from the skin. If the flexible arms are plastically deformable, they can then return back to (roughly) their original position, allowing for multiple uses of the device (either on the same medicament delivery device if the medicament delivery device is reusable, and/or on another medicament delivery device if the device is removable).

The device described herein can be considered to be a pain alleviation device, as it can alleviate injection pain. The device could be a medicament delivery add-on in its own right, as shown in the examples in the Figures, or it could be a medicament delivery device component, such as a medicament delivery member guard or a housing that includes the flexible arms described herein.

Various designs are possible for the attachment portion 12. For example, the attachment portion could attach with a friction lock (as shown in the examples in the Figures), or could have a clip, a lock, or another kind of attachment. The attachment portion could also be adhered to the medicament delivery device. The attachment could be permanent or removable - that is, the device could be designed to be removable or to be permanently attached to a medicament delivery device. Providing a removable attachment would allow for the attachment to be reused on another medicament delivery device.

In examples where the device is a medicament delivery device component, the attachment portion would attach the component to the rest of the medicament delivery device. The component could be a housing or a medicament delivery member guard, for example.

The attachment portion would typically be a distal portion of the device. The attachment portion is shown as a ring in the example in Figures 1 and 2, though it could have various other shapes. The attachment portion is shown with a circular cross-section perpendicular to the axis 20 in Figure 1, though it could have a different shaped cross-section such as a triangular, square or irregularly shaped cross-section. The attachment portion could also be flexible to allow attachment to different device diameters and shapes, which has the advantage of allowing the device to be used with various different medicament delivery devices. Alternatively, the attachment portion could be rigid; this could be beneficial in that it could limit use to a specific device or devices, thereby avoiding use of the device on the wrong type of medicament delivery device.

Various designs are also possible for the arms 14. Four arms are shown on the examples in the Figures, though 1, 2, 3 or more arms could alternatively be provided. The arms shown are all the same, though the arms could also differ from one another. The arms are shown tapering from a wider part at the end attached to the attachment portion (first end) down to a thinner part at the end distal from the attachment portion (second end), though the arms could also be the same width for their full length. The arms are shown tapering by variation in width in both a radial direction 25 relative to the axis and a circumferential direction relative to the axis, though the arms could alternatively only taper in the radial direction 25 or in the circumferential direction 26. The arms are shown as arc-shaped (describe an arc) when viewed from the side (i.e. the shape such as that shown for two of the arms in Figure 2., or in other words, when a cross-section along the perpendicular axis and through an arm is taken and viewed in a direction perpendicular to the axis 20, the arms are arc-shaped), though they could alternatively be another shape, such as straight. The second end is shown as being further from the axis than the first end, though this is also optional, and the arms could be parallel to the axis, for example.

Various options are also possible for the protrusions 18. The Figures show six protrusions on each arm, though more or fewer protrusions could be provided. The protrusions are shown evenly distributed along the length of the arms, though the protrusions could be unevenly spaced and/or only provided along part of the length of the arms. The protrusions are shown as a tapered spike with a sharp end, but could also have other shapes and/or could be blunt ended protrusions (e.g. a frustoconical shape or a cylindrical shape). Figure 2 shows an example with the protrusions all the same size, and Figure 3 shows longer protrusions closer to the first end of the flexible arm, with the protrusion length reducing as a function of distance from the first end of the flexible arm. Irregular patterns of protrusion length and width are also possible. The provision of protrusions in general is also optional.

The example shown in Figures 4 and 5 shows an autoinjector, but the device could be used on other medicament delivery devices such as pen injectors, for example. A suitable autoinjector could include features such as an outer housing, a medicament delivery member guard, a syringe comprising a medicament barrel and a medicament delivery member, and/or a powerpack, for example.

Various modifications to the embodiments described are possible and will occur to those skilled in the art without departing from the invention which is defined by the following claims.

## Claims

1. A device (10) for alleviating injection pain, the device (10) comprising
an attachment portion (12) for attachment to a medicament delivery device (30),
a plurality of flexible arms (14), each flexible arm (14) extending from a first end (16) attached to the attachment portion (12) to a second end (17) distal from the attachment portion (12) and comprising a plurality of protrusions (18) attached to the flexible arm (14),
wherein the second end (17) of each flexible arm (14) is further from an axis (20) than the first end (16) of the flexible arm (14) and the flexible arms (14) are arranged such that, as the device (10) is pressed against the skin, the flexible arms (14) flex outwards away from the axis (20) and the protrusions (18) rub across the skin, providing a sensation that can distract from injection pain.

2. The device (10) of claim 1, wherein the width of the second end (17) of each of the flexible arm (14) is thinner than the width of the first end (16) of the flexible arm (14).

3. The device (10) of claim 1 or 2, wherein the width of each of the flexible arms (14) tapers from the first end (16) to the second end (17).

4. The device (10) of any previous claim, wherein each of the flexible arms (14) is more flexible at the second end (17) than at the first end (16).

5. The device (10) of any previous claim, wherein the protrusions (18) are distributed along each of the flexible arms (14) between the first end (16) and the second end (17).

6. The device (10) of claim 5, wherein the protrusions (18) proximal to the first end (16) of the flexible arms (14) are longer than the protrusions (18) proximal to the second end (17) of the flexible arms (14).

7. The device (10) of claim 5 or 6, wherein the device (10) extends along the axis (20) from a proximal end (22) to a distal end (24), wherein the first ends (16) of the flexible arms (14) are closer to the distal end (24) than the second ends (17) of the flexible arms (14).

8. The device (10) of any previous claim, wherein each of the flexible arma (14) describes an arc.

9. The device (10) of any previous claim, wherein the device (10) is a medicament delivery device add-on and the attachment portion (12) is removably attachable to a medicament delivery device.

10. A medicament delivery device comprising the device (10) of any previous claim.

## Patentansprüche

1. Vorrichtung (10) zum Lindern von Injektionsschmerz, die Vorrichtung (10) umfassend
einen Befestigungsabschnitt (12) für Befestigung an einer Arzneimittelabgabevorrichtung (30),
eine Vielzahl von flexiblen Armen (14), wobei sich jeder flexible Arm (14) von einem ersten Ende (16), das an dem Befestigungsabschnitt (12) befestigt ist, zu einem zweiten Ende (17), distal von dem Befestigungsabschnitt (12), erstreckt, und umfassend eine Vielzahl von Vorsprüngen (18), die an dem flexiblen Arm (14) befestigt sind,
wobei das zweite Ende (17) jedes flexiblen Arms (14) weiter von einer Achse (20) als das erste Ende (16) des flexiblen Arms (14) ist und die flexiblen Arme (14) derart arrangiert sind, dass sich, wenn die Vorrichtung (10) gegen die Haut gedrückt wird, die flexiblen Arme (14) nach außen von der Achse (20) weg biegen und die Vorsprünge (18) über die Haut reiben, wobei eine Empfindung, die von Injektionsschmerz ablenken kann, bereitgestellt wird.

2. Vorrichtung (10) nach Anspruch 1, wobei die Breite des zweiten Endes (17) jedes der flexiblen Arme (14) dünner als die Breite des ersten Endes (16) des flexiblen Arms (14) ist.

3. Vorrichtung (10) nach Anspruch 1 oder 2, wobei sich die Breite jedes der flexiblen Arme (14) von dem ersten Ende (16) zu dem zweiten Ende (17) verjüngt.

4. Vorrichtung (10) nach einem der vorhergehenden Ansprüche, wobei jeder der flexiblen Arme (14) an dem zweiten Ende (17) flexibler als an dem ersten Ende (16) ist.

5. Vorrichtung (10) nach einem der vorhergehenden Ansprüche, wobei die Vorsprünge (18) entlang jedes der flexiblen Arme (14) zwischen dem ersten Ende (16) und dem zweiten Ende (17) verteilt sind.

6. Vorrichtung (10) nach Anspruch 5, wobei die Vorsprünge (18) proximal zu dem ersten Ende (16) der flexiblen Arme (14) länger als die Vorsprünge (18) proximal zu dem zweiten Ende (17) der flexiblen Arme (14) sind.

7. Vorrichtung (10) nach Anspruch 5 oder 6, wobei sich die Vorrichtung (10) entlang der Achse (20) von einem proximalen Ende (22) zu einem distalen Ende (24) erstreckt, wobei die ersten Enden (16) der flexiblen Arme (14) näher zu dem distalen Ende (24) als die zweiten Enden (17) der flexiblen Arme (14) sind.

8. Vorrichtung (10) nach einem der vorhergehenden Ansprüche, wobei jeder der flexiblen Arme (14) einen Bogen beschreibt.

9. Vorrichtung (10) nach einem der vorhergehenden Ansprüche, wobei die Vorrichtung (10) ein Arzneimittelabgabevorrichtungszusatz ist und der Befestigungsabschnitt (12) an einer Arzneimittelabgabevorrichtung lösbar befestigbar ist.

10. Arzneimittelabgabevorrichtung, umfassend die Vorrichtung (10) nach einem der vorhergehenden Ansprüche.

## Revendications

1. Dispositif (10) permettant d'atténuer une douleur d'injection, le dispositif (10) comprenant
une partie de fixation (12) pour fixation à un dispositif de délivrance de médicament (30),
une pluralité de bras flexibles (14), chaque bras flexible (14) s'étendant d'une première extrémité (16) fixée à la partie de fixation (12) à une seconde extrémité (17) distale par rapport à la partie de fixation (12) et comprenant une pluralité de parties saillantes (18) fixées au bras flexible (14),
dans lequel la seconde extrémité (17) de chaque bras flexible (14) est plus éloignée d'un axe (20) que la première extrémité (16) du bras flexible (14) et les bras flexibles (14) sont agencés de telle sorte que, à mesure que le dispositif (10) est pressé contre la peau, les bras flexibles (14) fléchissent vers l'extérieur à l'écart de l'axe (20) et les parties saillantes (18) frottent à travers la peau, fournissant une sensation qui peut distraire d'une douleur d'injection.

2. Dispositif (10) selon la revendication 1, dans lequel la largeur de la seconde extrémité (17) de chacun des bras flexibles (14) est plus mince que la largeur de la première extrémité (16) du bras flexible (14).

3. Dispositif (10) selon la revendication 1 ou 2, dans lequel la largeur de chacun des bras flexibles (14) s'effile de la première extrémité (16) à la seconde extrémité (17).

4. Dispositif (10) selon l'une quelconque revendication précédente, dans lequel chacun des bras flexibles (14) est plus flexible au niveau de la seconde extrémité (17) qu'au niveau de la première extrémité (16).

5. Dispositif (10) selon l'une quelconque revendication précédente, dans lequel les parties saillantes (18) sont réparties le long de chacun des bras flexibles (14) entre la première extrémité (16) et la seconde extrémité (17).

6. Dispositif (10) selon la revendication 5, dans lequel les parties saillantes (18) proximales par rapport à la première extrémité (16) des bras flexibles (14) sont plus longues que les parties saillantes (18) proximales par rapport à la seconde extrémité (17) des bras flexibles (14).

7. Dispositif (10) selon la revendication 5 ou 6, dans lequel le dispositif (10) s'étend le long de l'axe (20) d'une extrémité proximale (22) à une extrémité distale (24), dans lequel les premières extrémités (16) des bras flexibles (14) sont plus près de l'extrémité distale (24) que les secondes extrémités (17) des bras flexibles (14).

8. Dispositif (10) selon l'une quelconque revendication précédente, dans lequel chacun des bras flexibles (14) décrit un arc.

9. Dispositif (10) selon l'une quelconque revendication précédente, dans lequel le dispositif (10) est un composant additionnel de dispositif de délivrance de médicament et la partie de fixation (12) peut être fixée de façon amovible à un dispositif de délivrance de médicament.

10. Dispositif de délivrance de médicament comprenant le dispositif (10) selon l'une quelconque revendication précédente.
